# EUROPEAN PATENT APPLICATION

(11) **EP 4 177 341 A1**
(43) Date of publication of application: **10.05.2023**
(21) Application number: 21832413.5
(22) Date of filing: 02.07.2021
(51) Int. Cl.: C12N 5/0775, A61K 35/32, A61P 19/02

(54) **METHOD FOR PRODUCING SYNOVIAL MEMBRANE-DERIVED MESENCHYMAL STEM CELLS AND METHOD FOR PRODUCING CELL PREPARATION FOR JOINT TREATMENT**

(30) Priority: 03.07.2020 JP 2020115373; 24.03.2021 JP 2021049722
(71) Applicant: FUJIFILM Corporation, Tokyo 106-8620 (JP); National University Corporation Tokyo Medical and Dental University, Tokyo 113-8510 (JP)
(72) Inventor: SEKIYA, Ichiro, Tokyo 113-8510 (JP); MIZUNO, Mitsuru, Tokyo 113-8510 (JP); KATANO, Hisako, Tokyo 113-8510 (JP); OZEKI, Nobutake, Tokyo 113-8510 (JP); NAKAMURA, Kentaro, Ashigarakami-gun, Kanagawa 258-8577 (JP); YOSHIDA, Tomomi, Ashigarakami-gun, Kanagawa 258-8577 (JP)
(74) Representative: Hoffmann Eitle
(86) International application number: PCT/JP2021/025141
(87) International publication number: WO 2022/004878

(57) **Abstract**

An object of the present invention is to provide a production method for a synovium-derived mesenchymal stem cell, which makes it possible to obtain a sufficient amount of synovium-derived mesenchymal stem cells from the synovial tissue, a production method for a cell preparation for joint medical treatment using the production method for a synovium-derived mesenchymal stem cell, synovium-derived mesenchymal stem cell, and a cell preparation for a joint medical treatment. According to the present invention, there is provided a production method for a synovium-derived mesenchymal stem cell, which includes treating a synovial tissue with an enzyme for 2 hours or more; and washing a mixture after the enzyme treatment until a residual enzyme concentration in a supernatant is 0.5 ng/mL or less, to obtain a synovium-derived mesenchymal stem cell.

## Description

### Field of the Invention

The present invention relates to a method of producing a synovium-derived mesenchymal stem cell, which includes washing a mixture obtained by treating a synovial tissue with an enzyme for a predetermined time. The present invention further relates to a production method for a cell preparation for a joint medical treatment using the above method.

### Description of the Related Art

In the field of orthopedics, articular cartilage injury and meniscus injury are frequently seen as objects of daily medical practice and are widely recognized as diseases from which a large number of patients suffer. In a case where articular cartilage injury or meniscus injury occurs, arthralgia, a decrease in a mobile region, hydrarthrosis, dyskinesia, or the like occurs. A patient having traumatic articular cartilage injury or meniscus injury is usually treated medically by an orthopaedist. Surgical treatment for cartilage injury or meniscus injury aims at removing debris that causes further deterioration of the joint and restoring the function of the affected joint. However, it is generally known that cartilage and meniscus tissues are difficult to self-regenerate.

On the other hand, due to the recent progress in regenerative medicine technology, cell therapy capable of repairing cartilage or meniscus has become popular. Among the above, the mesenchymal stem cell (MSC) is expected as a cell source for a useful cell therapy. The mesenchymal stem cells can be collected from various body tissues and have been reported to be isolated from the bone marrow tissue (Non-Patent Document 1), the adipose tissue (Non-Patent Document 2), the muscle tissue (Non-Patent Document 3), the synovial tissue (Non-Patent Document 4), and the periosteal tissue (Non-Patent Document 5). In particular, it has been reported that the synovium-derived mesenchymal stem cell has high proliferation ability and high cartilage forming ability as compared with mesenchymal stem cells derived from various mesenchymal tissues such as bone marrow (Non-Patent Document 6). In addition, JP5928961B and JP5656183B disclose methods of medically treating articular cartilage injury and meniscus injury by using synovium-derived mesenchymal stem cells.

### Prior Art Documents

### Non-Patent Documents

Non-Patent Document 1: Prockop, D. J., 1997, Science. 276: 71-4
Non-Patent Document 2: Zuk, P. A. et al., 2002, Mol Biol Cell. 13: 4279-95
Non-Patent Document 3: Cao et al., 2003, Nat Cell Biol. 5: 640-6
Non-Patent Document 4: De Bari, C. et al., 2001, Arthritis Rheum. 44: 1928-42
Non-Patent Document 5: Fukumoto, T. et al., 2003, Osteoarthritis Cartilage. 11: 55-64
Non-Patent Document 6: Sakaguchi, et al., 2005, Arthritis Rhum. 52: 2521-9

### Patent Documents

Patent Document 1: JP5928961B
Patent Document 2: JP5656183B

### SUMMARY OF THE INVENTION

Patent Documents 1 and 2 disclose medical treatments using synovium-derived mesenchymal stem cells. On the other hand, in a process of actually producing cells, it was revealed that the production method disclosed above may not achieve a sufficient cell quantity enough to be subjected to an autologous medical treatment.

An object of the present invention is to provide a production method for a synovium-derived mesenchymal stem cell, which makes it possible to obtain a sufficient amount of synovium-derived mesenchymal stem cells from the synovial tissue. Further, an object of the present invention is to provide a production method for a cell preparation for a joint medical treatment using the production method for a synovium-derived mesenchymal stem cell. A further object of the present invention is to provide synovium-derived mesenchymal stem cell and a cell preparation for a joint medical treatment.

As a result of diligent studies to achieve the above object, the inventors of the present invention succeeded in producing a sufficient amount of synovium-derived mesenchymal stem cells, enough to be subjected to a medical treatment, by adjusting the time taken for enzymatically treating the synovial tissue and providing a step of washing cells. It is noted that it was conceived that increasing the enzyme treatment time of the synovial tissue should be avoided as much as possible since this increase leads to injury to the synovial tissue. In fact, in a case where the enzyme treatment time was changed from 1 hour to 1.5 hours, the cell quantity was significantly reduced, and the cell quantity could not be increased. On the other hand, it was found that the cell quantity can be unexpectedly increased in a case of deliberately providing a cell washing step which was not conceived to be necessary because the enzyme is generally inactivated by the autologous serum. The inventors of the present invention found that the effect of significantly increasing the cell quantity can be obtained for the first time by making two contrivances, that is, the extension of the enzyme treatment time and the inclusion of the cell washing step. The present invention has been completed based on the above findings.

That is, according to the present invention, the following inventions are provided.
<1> A production method for a synovium-derived mesenchymal stem cell, which is a production method for a synovium-derived mesenchymal stem cell from a synovial tissue, the method comprising:
   treating a synovial tissue with an enzyme for 2 hours or more; and
   washing a mixture after the enzyme treatment until a residual enzyme concentration in a supernatant is 0.5 ng/mL or less, to obtain a synovium-derived mesenchymal stem cell.
<2> The method according to <1>, in which the enzyme is a mixed enzyme including one or more kinds of collagenases and one or more kinds of neutral proteases.
<3> The method according to <1> or <2>, in which the enzyme is Liberase (registered trade name).
<4> The method according to any one of <1> to <3>, in which an enzyme concentration in the enzyme treatment is 0.01 mg/ml to 10 mg/ml.
<5> The method according to any one of <1> to <4>, in which the synovial tissue is derived from a human.
<6> The method according to any one of <1> to <5>, in which the synovial tissue is a synovial tissue derived from a single donor.
<7> The method according to any one of <1> to <6>, in which a subject from which synovium is collected and a subject to which the synovium-derived mesenchymal stem cell is transplanted are the same subject.
<8> The method according to any one of <1> to <7>, in which a mass ratio of the synovial tissue to the enzyme is 1,000:1 to 10:1.
<9> The method according to any one of <1> to <8>, in which the washing includes washing a plurality of times using a culture medium.
<10> The method according to <9>, in which the culture medium is an α-modified Eagle minimum essential medium.
<11> The method according to any one of <1> to <10>, further comprising culturing, for 10 days or more, the synovium-derived mesenchymal stem cell after the washing to proliferate the synovium-derived mesenchymal stem cell.
<12> The method according to <11>, in which the culturing for 10 days or more is carried out without culture medium exchange.
<13> The method according to <11> or <12>, in which the culturing for 10 days or more is carried out in a culture medium containing an autologous serum.
<14> The method according to any one of <1> to <13>, in which the synovium-derived mesenchymal stem cell is produced without being co-cultured with a cell other than the synovium-derived mesenchymal stem cell.
<15> A production method for a cell preparation for a joint medical treatment, the production method comprising:
   producing a synovium-derived mesenchymal stem cell by the method according to any one of <1> to <14>; and
   producing a cell preparation for a joint medical treatment using the synovium-derived mesenchymal stem cell.
<16> The production method for a cell preparation for a joint medical treatment according to <15>, in which the joint medical treatment is a medical treatment for a meniscus injury.
<17> Synovium-derived mesenchymal stem cell produced by the method according to any one of <1> to <14>.
<18> A cell preparation for a joint medical treatment, produced by the method according to <15> or <16>.

<A> Medical treatment method for a joint, comprising:
a step of transplanting synovium-derived mesenchymal stem cell produced by a method according to an aspect of the present invention so that a cartilage injury part or a meniscus injury part is covered with the synovium-derived mesenchymal stem cell; and
a step of differentiating the synovium-derived mesenchymal stem cell into a cartilage cell to regenerate cartilage tissue in situ at a cartilage injury part or a meniscus injury part.

According to the present invention, a sufficient amount of synovium-derived mesenchymal stem cells can be obtained from the synovial tissue.

### DESCRIPTION OF THE PREFERRED EMBODIMENTS

Hereinafter, embodiments for carrying out the present invention will be described in detail.

### [Production method for synovium-derived mesenchymal stem cell]

A production method for a synovium-derived mesenchymal stem cell according to an embodiment of the present invention is a method of producing a synovium-derived mesenchymal stem cell from a synovial tissue, where the method includes treating a synovial tissue with an enzyme for 2 hours or more; and washing a mixture after the enzyme treatment until a residual enzyme concentration in a supernatant is 0.5 ng/mL or less, to obtain a synovium-derived mesenchymal stem cell.

### <Synovial tissue>

The synovial tissue can be collected from the unloaded portion of the joint under anesthesia.

The biological origin of the synovial tissue is not particularly limited, and a synovial tissue derived from any biological material, preferably a mammal, can be used. For example, a synovial tissue derived from a primate (for example, a chimpanzee, a Japanese monkey, or a human) can be used, and a human-derived synovial tissue can be used particularly preferably.

The synovial tissue may be a synovial tissue derived from a single donor or may be a synovial tissue derived from a plurality of donors; however, it is preferably a synovial tissue derived from a single donor.

In a case of producing a synovium-derived mesenchymal stem cell for the intended purpose of administration to a human, it is preferable to use a synovial tissue collected from a donor of which histocompatibility antigen type is identical or similar to that of the recipient. More preferably, a subject from which the synovium is collected and a subject to which the synovium-derived mesenchymal stem cell is transplanted are the same subject. That is, it is preferable to use the synovial tissue collected from the recipient itself (autologous transplantation).

The amount of the synovial tissue to be collected can be determined in consideration of the type of donor or the required amount of synovium-derived mesenchymal stem cells. For example, it is possible to obtain synovium-derived mesenchymal stem cells from the synovial tissue of 0.1 g to 10 g, preferably 0.1 g to 2.0 g, more preferably 0.1 g to 1.5 g, and still more preferably 0.1 g to 1.0 g. The collected synovial tissue is shredded with scissors or the like as necessary, and then subjected to the enzyme treatment described below.

### <Treatment with enzyme>

In the present invention, the synovial tissue is treated with an enzyme for 2 hours or more.

The enzyme is not particularly limited as long as it is an enzyme including a protease. However, it is preferably a mixed enzyme including one or more kinds of collagenases and one or more kinds of neutral proteases. A particularly preferred enzyme is Liberase (registered trade name). As the Liberase (registered trade name), it is possible to use, for example, Liberase (registered trade name) MNP-S (manufactured by F. Hoffmann-La Roche, Ltd.), which is an enzyme including a collagenase class I, a collagenase class II, and a neutral protease (thermolysin).

The enzymatic reaction can be carried out in an aqueous solution containing an enzyme, and an aqueous solution containing human serum may be used. The human serum may be an autologous serum or an allogeneic serum; however, it is preferably an autologous serum.

The enzyme concentration in the enzyme treatment is preferably 0.01 mg/ml to 10 mg/ml, more preferably 0.1 mg/ml to 10 mg/ml, still more preferably 0.5 mg/ml to 10 mg/ml, ever still more preferably 0.5 mg/ml to 5.0 mg/ml, particularly preferably 0.5 mg/ml to 2.0 mg/ml, and most preferably 0.7 mg/ml to 2.0 mg/ml.

The mass ratio of the synovial tissue to the enzyme is preferably 1,000:1 to 10:1, more preferably 500:1 to 20:1, and still more preferably 200:1 to 40:1.

The enzymatic reaction can be carried out at a temperature of preferably 15°C to 40°C, more preferably 20°C to 35°C, and still more preferably 25°C to 35°C.

The reaction time may be 2 hours or more, more preferably 2.5 hours or more, and still more preferably 3 hours or more. The upper limit of the reaction time is not particularly limited; however, it may be 10 hours or less, 9 hours or less, 8 hours or less, 7 hours or less, 6 hours or less, 5 hours or less, or 4 hours or less.

The enzyme-treated mixture contains synovium-derived mesenchymal stem cells.

The enzyme-treated mixture is transferred to a centrifuge tube through a cell strainer and centrifuged, whereby synovium-derived mesenchymal stem cells can be recovered.

### <Washing>

In the present invention, the mixture after the enzyme treatment is washed until the residual enzyme concentration in the supernatant is 0.5 ng/mL or less. The residual enzyme concentration in the supernatant is preferably 0.3 ng/mL or less, more preferably 0.2 ng/mL or less, and particularly preferably 0.1 ng/mL or less.

The washing can be carried out by resuspending the synovium-derived mesenchymal stem cells recovered with the above-described centrifugation treatment in a culture medium and carrying out centrifugation again (for example, at 400 g for 5 minutes). As the culture medium, α-modified Eagle minimum essential medium (αMEM) can be used, which is not particularly limited. The washing may be carried out a plurality of times (two times or more) using the culture medium as described above.

### <Proliferation>

In the present invention, the synovium-derived mesenchymal stem cells after the washing may be cultured for 10 days or more to proliferate the synovium-derived mesenchymal stem cells.

As the culture medium that is used in the above-described culture, a culture medium that is used for the general culture of animal cells can be prepared as a basal medium. Examples of the culture medium that is used for general culture of animal cells include αMEM, a Dulbecco's modified Eagle medium (DMEM), a mixed medium of DMEM and F12 (DMEM:F12 = 1:1), an RPMI medium (a GIBCO (registered trade name) RPMI 1640 medium or the like), and a mixed medium of DMEM/F12 and RPMI (DMEM/F12:RPMI = 1:1), which are not particularly limited.

The culture medium may be a culture medium containing serum or may be a culture medium not containing serum. In a case of producing a synovium-derived mesenchymal stem cells from the autologous tissue for the intended purpose of administration to a living body, the culture medium may contain a serum of origin of the same species. That is, in a case of producing a synovium-derived mesenchymal stem cells from a human tissue for the intended purpose of administration to a human, a culture medium containing a human serum may be used. In a case where serum is used, the serum may be an autologous serum or may be an allogeneic serum; however, it is preferably an autologous serum. In a case where serum is used, the adding amount of serum in the culture medium is, for example, 20% by volume or less, 10% by volume or less, or 5% by volume or less.

The cell culture conditions are not particularly limited, and general cell culture conditions can be employed. Examples thereof include culture at a temperature of 30°C to 40°C and 3% to 7% CO₂, which are not particularly limited. As one example, culture at a temperature of 37°C and 5% CO₂ can be mentioned.

In the present invention, it is preferable to carry out the above-described culturing for 10 days or more without culture medium exchange. In addition, in the above-described culturing for 10 days or more, it is preferable that the synovium-derived mesenchymal stem cells are produced without being co-cultured with cells other than the synovium-derived mesenchymal stem cells.

The differentiation of synovium-derived mesenchymal stem cells into cartilage cells progresses as the culture period increases, and thus, it is known that the in situ cartilage forming ability of synovium-derived mesenchymal stem cells decreases when the culture period exceeds a specific length. Therefore, in the present invention, it is preferable to adjust the culture period in order to proliferate the synovium-derived mesenchymal stem cells in an undifferentiated state and in a state having a good in situ cartilage forming ability. In addition, in the present invention, it is necessary to consider the need to prepare a sufficient number of undifferentiated synovial stem cells in order to cover the cartilage injury part and regenerate the affected part. As a result, the culture period is preferably 5 days or more, 7 days or more, and 10 days or more, more preferably 10 to 14 days, 10 to 21 days, or 10 to 28 days, and still more preferably 10 to 21 days.

It is known that mesenchymal stem cells are differentiated into cartilage cells by culturing them in a cartilage forming culture medium to which transforming growth factor β3 (TGF-β3), dexamethasone, and bone morphogenetic protein 2 (BMP-2) have been added, whereby the cartilage tissue can be prepared in vitro. As a result, in the present invention, the synovium-derived mesenchymal stem cells isolated are preferably cultured in the absence of TGF-β3, dexamethasone, or BMP-2 in order to prevent the synovium-derived mesenchymal stem cells from being differentiated into cartilage cells.

It is also known that in the synovium-derived mesenchymal stem cells, the in situ cartilage forming ability decreases in inverse proportion to the number of passages of the mesenchymal stem cells in vitro. As a result, it is preferable to produce synovium-derived mesenchymal stem cells in the primary culture or first subculture in order to prepare undifferentiated mesenchymal stem cells,

In the present invention, the serum that is used in an autologous medical treatment is of autologous origin, the amount of serum that can be collected from the donor in an autologous medical treatment is limited, and the cell density is required to be equal to or higher than a certain level from the viewpoint of the proliferation of synovium-derived stem cells. For these reasons, it is preferable that the synovium-derived mesenchymal stem cells after the enzyme treatment are seeded at a cell number of 100 cells/cm² or more and 5,000 cells/cm² or less, 200 cells/cm² or more and 5,000 cells/cm² or less, 500 cells/cm² or more and 5,000 cells/cm² or less, 500 cells/cm² or more and 2,500 cells/cm² or less, or 500 cells/cm² or more and 2,000 cells/cm² or less, and then cultured. In addition, in order to proliferate the synovium-derived mesenchymal stem cells after the enzyme treatment, it is more preferable to culture the cells for 10 days or more.

The number of cells obtained at the end of the culture is preferably 1.0 × 10⁷ cells or more, 2.0 × 10⁷ cells or more, 2.5 × 10⁷ cells or more, or 3.0 × 10⁷ cells or more, more preferably 4.0 × 10⁷ cells or more, still more preferably 5.0 × 10⁷ cells or more, and particularly preferably 6.0 × 10⁷ cells or more.

### <Synovium-derived mesenchymal stem cell>

The mesenchymal stem cell is a somatic stem cell derived from a mesodermal tissue (mesenchyme). The mesenchymal stem cells are known to be present in bone marrow, synovium, periosteum, adipose tissue, and muscle tissue, and they are known to have the ability to be differentiated into osteoblasts, cartilage cells, adipose cells, and muscle cells. In relation to the differentiation of mesenchymal stem cells into cartilage cells, it is known that the addition of BMP or TGF-β to the culture solution promotes the differentiation of undifferentiated mesenchymal stem cells into cartilage cells, and the cartilage tissue can be regenerated under in vitro conditions.

The mesenchymal stem cell can be confirmed by detecting a molecule characteristic of the mesenchymal stem cell, for example, an enzyme, a receptor, a low-molecular-weight compound, or the like. Examples of the molecule characteristic of mesenchymal stem cells include, which are not limited to, CD73, CD90, CD105, and CD166, which are cell surface markers (positive markers). In addition, examples of the negative marker that are not expressed in mesenchymal stem cells include, but are not limited to, CD19, CD34, CD45, HLA-DR, CD11b, and CD14. It is noted that CD is an abbreviation for Clusters of diffusion, and HLA-DR is an abbreviation for human leukocyte engine-D-related. These positive markers and negative markers can be used to confirm that a cell is the mesenchymal stem cell. Although an immunological method can be used for the detection of these markers, the detection may be carried out by quantifying the mRNA amount of each molecule.

In the present specification, the synovium-derived mesenchymal stem cell is a stem cell contained in the synovium. The synovium-derived mesenchymal stem cell is a kind of mesenchymal stem cell. The synovium-derived mesenchymal stem cell can be detected, for example, by detecting CD90 positivity, CD45 negativity, or cartilage differentiation potency; however, the detection method is not particularly limited.

According to the present invention, there is provided synovium-derived mesenchymal stem cell produced by the method of the present invention.

The synovium-derived mesenchymal stem cell produced by the method according to the embodiment of the present invention can be used as a cell preparation for a joint medical treatment. Further, the synovium-derived mesenchymal stem cell produced by the method according to the embodiment of the present invention can be also used, for example, for research related to differentiation of mesenchymal stem cells, pharmaceutical screening for various diseases, evaluation of efficacy and safety of pharmaceutical candidate compounds, and the like.

### [Production method for cell preparation for joint medical treatment]

The present invention relates to a production method for a cell preparation for a joint medical treatment, which includes;
producing a synovium-derived mesenchymal stem cell by the above-described method according to the embodiment of the present invention, and
producing a cell preparation for a joint medical treatment using the synovium-derived mesenchymal stem cell.

According to the present invention, there is provided a cell preparation for a joint medical treatment, produced by the above-described production method for a cell preparation for a joint medical treatment.

That is, according to the present invention, it is possible to produce a cell preparation for a joint medical treatment, containing the synovium-derived mesenchymal stem cell obtained by the method according to the embodiment of the present invention as an active ingredient.

Examples of the joint medical treatment include a medical treatment for a disease associated with injury, damage, or inflammation of the joint, and examples of the medical treatment include a medical treatment for a joint disease resulting from degeneration and/or inflammation of the connective tissue such as cartilage, or a non-inflammatory joint disease. Examples of the joint medical treatment include a medical treatment for a disease selected from the group consisting of meniscus injury, traumatic cartilage injury, osteochondritis dissecans, aseptic osteonecrosis, osteoarthritis (for example, knee osteoarthritis), rheumatoid arthritis (for example, chronic rheumatoid arthritis), gout, reactive arthritis, psoriatic arthritis, juvenile arthritis, inflammatory arthritis, and articular cartilage defect, which are not limited to these diseases.

In a case where the synovium-derived mesenchymal stem cell produced by the method according to the embodiment of the present invention is used as a cell preparation for a joint medical treatment, this cell may be made into a pharmaceutical preparation in a form suitable for administration to an individual, by being mixed with a pharmaceutically acceptable carrier according to a conventional method. Examples of the carrier include physiological saline and distilled water for injection, which has been made to be isotonic by adding glucose and other auxiliary agents (for example, D-sorbitol, D-mannitol, sodium chloride, and the like). Further, a buffering agent (for example, a phosphate buffer solution or a sodium acetate buffer solution), a soothing agent (for example, benzalkonium chloride, procaine hydrochloride, or the like), a stabilizer (for example, human serum albumin, polyethylene glycol, or the like), a storage agent, an antioxidant, and the like may be blended.

### [Medical treatment method for joint]

The present invention further relates to a medical treatment method for a joint. More specifically, the present invention relates to a medical treatment method for a disease selected from the group consisting of meniscus injury, traumatic cartilage injury, osteochondritis dissecans, aseptic osteonecrosis, osteoarthritis (for example, knee osteoarthritis), rheumatoid arthritis (for example, chronic rheumatoid arthritis), gout, reactive arthritis, psoriatic arthritis, juvenile arthritis, inflammatory arthritis, and articular cartilage defect.

The medical treatment method for a joint according to the embodiment of the present invention includes a step of transplanting the synovium-derived mesenchymal stem cell produced by the method according to the embodiment of the present invention so that a cartilage injury part or a meniscus injury part is covered with the synovium-derived mesenchymal stem cell; and a step of differentiating the synovium-derived mesenchymal stem cell into a cartilage cell to regenerate cartilage tissue in situ at a cartilage injury part or a meniscus injury part.

In a case where the synovium-derived mesenchymal stem cells produced by the method according to the embodiment of the present invention are transplanted into a patient, it is more preferable to apply, per cartilage injury part or meniscus injury part, synovium-derived mesenchymal stem cells of 2.0 × 10⁷ to 1.0 × 10¹¹ cells, 2.5 × 10⁷ to 1.0 × 10¹¹ cells, 3.0 × 10⁷ to 1.0 × 10¹¹ cells, 4.0 × 10⁷ to 1.0 × 10¹¹ cells, 2.5 × 10⁷ to 1.0 × 10¹⁰ cells, 2.5 × 10⁷ to 1.0 × 10⁹ cells, 2.5 × 10⁷ to 1.0 × 10⁸ cells, or 2.0 × 10⁷ to 1.0 × 10⁸ cells, or synovium-derived mesenchymal stem cells of 2.0 × 10⁷ to 1.0 × 10⁸ cells, in order to efficiently treat medically the cartilage injury part or the meniscus injury part.

By transplanting the synovium-derived mesenchymal stem cells into the cartilage injury part or the meniscus injury part, the cartilage injury part or the meniscus injury part is covered with the synovium-derived mesenchymal stem cells. The transplantation of the /synovium-derived mesenchymal stem cell can be carried out by open surgery or by arthroscopic surgery. In order to minimize the invasion, it is preferable to arthroscopically transplant the synovium-derived mesenchymal stem cell.

The cartilage injury part or the meniscus injury part may be covered with a suspension of synovium-derived mesenchymal stem cells or may be covered with a cell sheet of synovium-derived mesenchymal stem cells. For example, a gel having bioabsorbability such as gelatin or collagen can be used as a gel-like substance. The synovium-derived mesenchymal stem cell has a high ability to adhere to the cartilage-injury part or the meniscus injury part.

In a case of a medical treatment for cartilage injury, the minimally invasive method according to the embodiment of the present invention is characterized in that the cartilage injury part is covered with synovium-derived mesenchymal stem cells, and it includes the following steps:
holding the body position so that the cartilage injury part faces upward;
placing a cell sheet of synovium-derived mesenchymal stem cells, a suspension of synovium-derived mesenchymal stem cells, or a gel-like substance containing synovium-derived mesenchymal stem cells on the surface of the cartilage injury part; and
holding the body position for a specific period of time to allow the synovium-derived mesenchymal stem cells to adhere to the surface of the cartilage injury part.

In a case of a medical treatment for meniscus injury, the minimally invasive method according to the embodiment of the present invention is characterized in that the meniscus injury part is covered with synovium-derived mesenchymal stem cells, and it includes the following steps:
holding the body position so that the meniscus injury part faces downward;
injecting the suspension of synovium-derived mesenchymal stem cells into a knee j oint; and
holding the body position for a specific period of time to allow the synovium-derived mesenchymal stem cells to adhere to the surface of the meniscus injury part.

In order to reliably allow the synovium-derived mesenchymal stem cells to adhere to the surface of the cartilage-injury part or meniscus injury part, the transplanted synovium-derived mesenchymal stem cells are preferably held on the surface of the cartilage injury part or meniscus injury part for at least 10 minutes and preferably 15 minutes. In order to achieve this, the body position is held for at least 10 minutes and preferably 15 minutes in order to achieve that the cartilage injury part or the meniscus injury part faces upward and that the synovium-derived mesenchymal stem cells are held in the cartilage injury part or the meniscus injury part, which faces upward.

In order to further strengthen the adhesion of the synovium-derived mesenchymal stem cells to the cartilage-injury part or the meniscus injury part, the cartilage injury part or the meniscus injury part associated with the synovium-derived mesenchymal stem cells can be covered with the periosteum. The operation is completed after holding the synovium-derived mesenchymal stem cells on the surface of the cartilage injury part or the surface of the meniscus injury part for at least 10 minutes.

In the present invention, the transplanted synovium-derived mesenchymal stem cells are differentiated into cartilage cells at the cartilage-injury part or the meniscus injury part and the cartilage tissue is regenerated in situ at the cartilage-injury part or the meniscus injury part.

During the in situ cartilage formation process of the synovium-derived mesenchymal stem cells, the cartilage tissue is regenerated depending on the local microenvironment (the nutrient supply, the cytokine environment, and the like), and thus no external operation is required. As a result of the in situ cartilage formation of synovium-derived mesenchymal stem cells, the cartilage tissue is regenerated at the cartilage injury part or the meniscus injury part to repair the injury. Then, in a case of cartilage injury, a bone region, a boundary between the cartilage and the bone, a central part of the cartilage, a surface region, and a region adjacent to the original cartilage are formed as the original cartilage tissue, or in a case of the meniscus injury, meniscus cartilage is formed.

The present invention will be further specifically described with reference to Examples; however, the present invention is not limited by Examples.

### Examples

### <Comparative Example 1>

### Production of synovium-derived mesenchymal stem cell according to general production step (enzyme treatment time: 1 hour, without washing step)

Using a human specimen, synovium-derived mesenchymal stem cells were prepared from a synovial tissue according to a general production method. Specifically, a C1 specimen (0.5 g of the synovial tissue) was shredded with scissors and immersed in 5.0 mL of a Liberase aqueous solution. It is noted that as the Liberase aqueous solution, a solution obtained by dissolving 5.0 mg of Liberase MNP-S (manufactured by F. Hoffmann-La Roche, Ltd.) in 5.0 mL of water for injection containing human autologous serum of a final concentration of 20% was used. The enzymatic reaction was carried out at room temperature of 25°C for 1 hour. Then, the digested tissue solution was transferred to a 50 mL centrifuge tube through a cell strainer and centrifuged at 400 g for 5 minutes. The supernatant was removed, the obtained cell-concentrated suspension was suspended in a culture medium, and the entire amount thereof was seeded in a flask (seed density: 200 cells/cm²). It is noted that as the culture medium, a culture medium obtained by dissolving, in αMEM, human autologous serum to a final concentration of 10% was used. The culture was carried out in a CO₂ incubator (37°C, 5% CO₂), and after 2 weeks, the cells were recovered from the flask to produce synovium-derived mesenchymal stem cells. It is noted that a feature of the present cell production is that the culture medium is not exchanged during the culture period. The cell quantity finally obtained in this way was 1.8 × 10⁷ cells for C1. A summary of the results is shown in Table 1.

### <Comparative Example 2>

Production of synovium-derived mesenchymal stem cell according to production step in which enzyme treatment time is extended (enzyme treatment time: 1.5 hours, without washing step)

Using a human specimen, synovium-derived mesenchymal stem cells were prepared from a synovial tissue under the condition in which the enzyme treatment time was extended by 30 minutes as compared with the general production method. Specifically, a C2 specimen (0.9 g of the synovial tissue) was shredded with scissors and immersed in 5.0 mL of a Liberase aqueous solution. It is noted that as the Liberase aqueous solution, a solution obtained by dissolving 5.0 mg of Liberase MNP-S (manufactured by F. Hoffmann-La Roche, Ltd.) in 5.0 mL of water for injection containing human autologous serum of a final concentration of 20% was used. The enzymatic reaction was carried out at room temperature of 25°C for 1.5 hours. Then, the digested tissue solution was transferred to a 50 mL centrifuge tube through a cell strainer and centrifuged at 400 g for 5 minutes. The supernatant was removed, the obtained cell-concentrated suspension was suspended in a culture medium, and the entire amount thereof was seeded in a flask (seed density: 1,880 cells/cm²). It is noted that as the culture medium, a culture medium obtained by dissolving, in αMEM, human autologous serum to a final concentration of 10% was used. The culture was carried out in a CO₂ incubator (37°C, 5% CO₂), and after 2 weeks, the cells were recovered from the flask to produce synovium-derived mesenchymal stem cells. It is noted that a feature of the present cell production is that the culture medium is not exchanged during the culture period. The cell quantity finally obtained in this way was 0.8 × 10⁷ cells as C2. This was less than 1.0 × 10⁷ cells considered to be necessary for the medical treatment, which was at a level in which a cell amount required for the medical treatment could not be provided. A summary of the results is shown in Table 1.

### <Example 1>

Production of synovium-derived mesenchymal stem cell according to production step in which enzyme treatment time is extended and enzyme washing step is provided (enzyme treatment time: 3 hours, with washing step)

Using a human specimen, synovium-derived mesenchymal stem cells were prepared from a synovial tissue under the condition in which the enzyme treatment time was extended by 2 hours as compared with the general production method. Specifically, a J1 specimen (synovial tissue: 0.8 g), a J2 specimen (synovial tissue: 0.6 g), a J3 specimen (synovial tissue: 0.7 g), a J4 specimen (synovial tissue: 0.5 g), and a J5 specimen (0.3 g of synovial tissue) were each shredded with scissors and immersed in 5.0 mL of a Liberase aqueous solution. It is noted that as the Liberase aqueous solution, a solution obtained by dissolving 5.0 mg of Liberase MNP-S (manufactured by F. Hoffmann-La Roche, Ltd.) in 5.0 mL of water for injection containing human autologous serum of a final concentration of 20% was used. The enzymatic reaction was carried out at room temperature of 25°C for 3 hours. Then, the digested tissue solution was transferred to a 50 mL centrifuge tube through a cell strainer and centrifuged at 400 g for 5 minutes.

Then, as the washing step, a step of discarding the supernatant, carrying out resuspension with 20 mL of αMEM, and carrying out centrifugation at 400 g for 5 minutes was further provided. This washing step was repeated twice in total.

Then, the supernatant was removed, the obtained cell-concentrated suspension was suspended in a culture medium, and the entire amount was seeded in a flask (seed density: 1,874 cells/cm² for J1, 1,059 cells/cm² for J2, 1,987 cells/cm² for J3, and 1,513 cells/cm² for J4). It is noted that as the culture medium, a culture medium obtained by dissolving, in αMEM, human autologous serum to a final concentration of 10% was used. The culture was carried out in a CO₂ incubator (37°C, 5% CO₂), and after 2 weeks, the cells were recovered from the flask to produce synovium-derived mesenchymal stem cells. It is noted that a feature of the present cell production is that the culture medium is not exchanged during the culture period.

The cell quantities finally obtained in this way were 4.9 × 10⁷ cells for J1, 7.6 × 10⁷ cells for J2, 5.8 × 10⁷ cells for J3, 9.0 × 10⁷ cells for J4, and 6.5 × 10⁷ cells for J5. This significantly exceeded 1.0 × 10⁷ cells considered to be necessary for the medical treatment, which was at a level in which a cell amount required for the medical treatment could be sufficiently and stably provided. A summary of the results is shown in Table 1.

### <Example 2>

### Quantification of residual enzyme Liberase

In the production steps carried out in Comparative Example 1, Comparative Example 2, and Example 1, the amount of Liberase remaining in the culture medium was quantified. As a result, it was revealed that C1 is 10.2 ng/mL, C2 is 59.8 ng/mL, and J1 to J5 are all 0.1 ng/mL or less (equal to or lower than the detection limit of the kit). The results are shown in Table 1.

### <Example 3> Checking test of mesenchymal stem cell marker

The synovium-derived mesenchymal stem cells prepared in Comparative Examples 1 and 2 and Example 1 were subjected to a checking test for the expression of the mesenchymal stem cell markers. As a result, it was confirmed that all of the cells exhibit CD90 positivity, CD45 negativity, and cartilage differentiation potency and thus are synovium-derived mesenchymal stem cells.

**[Table 1]**

| | Comparative Example | | Example | | | | |
|---|---|---|---|---|---|---|---|
| Tissue code | C1 | C2 | J1 | J2 | J3 | J4 | J5 |
| Weight of synovial tissue (g) | 0.5 | 0.9 | 0.8 | 0.6 | 0.7 | 0.5 | 0.3 |
| Enzyme concentration (mg/mL) | 1 | 1 | 1 | 1 | 1 | 1 | 1 |
| Mass ratio of synovial tissue to enzyme | 100:1 | 180:1 | 160:1 | 120:1 | 140:1 | 100:1 | 60:1 |
| Enzyme treatment time (hour, minute) | 1:00 | 1:30 | 3:00 | 3:00 | 3:00 | 3:00 | 3:00 |
| Washing before seeding | Supernatant removal only | Supernatant removal only | Two times of centrifugal washing of αMEM (20mL) after supernatant removal | Two times of centrifugal washing of αMEM (20mL) after supernatant removal | Two times of centrifugal washing of αMEM (20mL) after supernatant removal | Two times of centrifugal washing of αMEM (20mL) after supernatant removal | Two times of centrifugal washing of αMEM (20mL) after supernatant removal |
| Cell quantity (×10⁶ cells) | 18.0 | 8.0 | 49.0 | 76.0 | 58.0 | 90.0 | 65.0 |
| Residual Liberase concentration (ng/mL) | 10.2 | 59.8 | < 0.1 | < 0.1 | < 0.1 | < 0.1 | < 0.1 |

### <Interpretation of results>

From the results shown in Table 1, it was revealed that the weight of the synovial tissue is not the dominant parameter with respect to the cell quantity. In addition, as it was conceived that extending the enzyme treatment time should be avoided since it causes injury to the synovial tissue, it was found that in C2 in which the enzyme treatment time was extended by 30 minutes with respect to C1, the cell quantity is actually reduced significantly. On the other hand, it was revealed that in a case of carrying out a combination in which the washing step, which has been conceived not always necessary since the serum is present in the culture medium, is deliberately provided and the enzyme treatment time is further extended, it is possible to achieve a significant increase in cell quantity as in J1 to J5. It was found that the above-described two contrivances, which have not been incorporated so far, are very important in the step of producing cells from the synovial tissue. In addition, the cell quantities of J1 to J5 obtained according to the above-described production were significantly exceeded 1.0 × 10⁷ cells considered to be necessary for the medical treatment, and this significantly exceeded the cell amount (2.0 × 10⁷ cells) capable of exhibiting the effect even in a case of allogeneic cells, and thus it was conceived to have remarkable value as a method of producing a synovium-derived mesenchymal stem cell assuming autologous cell therapy.

### <Example 4>

The synovium-derived mesenchymal stem cells produced as J1 to J5 were actually produced from specimens of meniscus injury patients themselves and, after the production, respectively administered to the meniscus injury patients as autologous cell therapy. As a result, favorable symptom relief was observed until 52 weeks after the administration in all the meniscus injury patients to which the synovium-derived mesenchymal stem cells produced as J1 to J5 were respectively administered. An improvement of 22 points or more was confirmed in terms of the Lysholm score (see Table 2 below), which is known as a symptom score, until 52 weeks after the administration compared with that before the treatment, and the reposition was also confirmed in the meniscus checking by magnetic resonance imaging (MRI), which indicated the medical treatment results were favorable.

**[Table 2]**

| Lysholm score: Evaluation carried out by person in charge of evaluation | | |
|---|---|---|
| Evaluation item | | Score |
| Limping (foot limping) (5 points) | Absent | 5 |
| | Seldom or often | 3 |
| | Always severe | 0 |
| Walking stability (30 points) | Walking is possible without problem | 30 |
| | Knees rarely collapse during sports or other vigorous exercise | 25 |
| | Knees frequently collapse during sports or other vigorous exercise | 15 |
| | Unstable at times in everyday life | 10 |
| | Unstable frequently in everyday life | 5 |
| | Always unstable when walking | 0 |
| Supporting ability (5 points) | Fully loadable | 5 |
| | Stick or crutch is necessary | 3 |
| | Loading application is impossible | 0 |
| Swelling (10 points) | Absent | 10 |
| | Associated with Knee collapse | 7 |
| | Due to vigorous exercise | 5 |
| | With exercise in everyday life | 2 |
| | Always | 0 |
| Going up and down stairs (5 points) | No problem | 5 |
| | Some problems | 3 |
| | Going up and down is possible only step by step | 2 |
| | Impossible | 0 |
| Pain (30 points) | Absent | 30 |
| | Occasionally slight during vigorous exercise | 25 |
| | At time when knees collapse | 20 |
| | During vigorous exercise | 15 |
| | During or after walking 2 km or more | 10 |
| | During or after walking less than 2 km | 5 |
| | Always | 0 |
| Bending and stretching of knee (5 points) | No problem | 5 |
| | Some problems | 3 |
| | Up to 90 degrees | 2 |
| | Impossible | 0 |
| Femur atrophy (5 points) | Absent | 5 |
| | Atrophy of 1 to 2 cm | 3 |
| | Atrophy of 2 cm | 0 |

## Claims

1. A production method for a synovium-derived mesenchymal stem cell, which is a production method for a synovium-derived mesenchymal stem cell from a synovial tissue, the method comprising:
treating a synovial tissue with an enzyme for 2 hours or more; and
washing a mixture after the enzyme treatment until a residual enzyme concentration in a supernatant is 0.5 ng/mL or less, to obtain a synovium-derived mesenchymal stem cell.

2. The method according to claim 1,
wherein the enzyme is a mixed enzyme including one or more kinds of collagenases and one or more kinds of neutral proteases.

3. The method according to claim 1 or 2,
wherein the enzyme is Liberase (registered trade name).

4. The method according to any one of claims 1 to 3,
wherein an enzyme concentration in the enzyme treatment is 0.01 mg/ml to 10 mg/ml.

5. The method according to any one of claims 1 to 4,
wherein the synovial tissue is derived from a human.

6. The method according to any one of claims 1 to 5,
wherein the synovial tissue is a synovial tissue derived from a single donor.

7. The method according to any one of claims 1 to 6,
wherein a subject from which synovium is collected and a subject to which the synovium-derived mesenchymal stem cell is transplanted are the same subject.

8. The method according to any one of claims 1 to 7,
wherein a mass ratio of the synovial tissue to the enzyme is 1,000:1 to 10:1.

9. The method according to any one of claims 1 to 8,
wherein the washing includes washing a plurality of times using a culture medium.

10. The method according to claim 9,
wherein the culture medium is an α-modified Eagle minimum essential medium.

11. The method according to any one of claims 1 to 10, further comprising:
culturing, for 10 days or more, the synovium-derived mesenchymal stem cell after the washing to proliferate the synovium-derived mesenchymal stem cell.

12. The method according to claim 11,
wherein the culturing for 10 days or more is carried out without culture medium exchange.

13. The method according to claim 11 or 12,
wherein the culturing for 10 days or more is carried out in a culture medium containing an autologous serum.

14. The method according to any one of claims 1 to 13,
wherein the synovium-derived mesenchymal stem cell is produced without being co-cultured with a cell other than the synovium-derived mesenchymal stem cell.

15. A production method for a cell preparation for a joint medical treatment, the production method comprising:
producing a synovium-derived mesenchymal stem cell by the method according to any one of claims 1 to 14; and
producing a cell preparation for a joint medical treatment using the synovium-derived mesenchymal stem cell.

16. The production method for a cell preparation for a joint medical treatment according to claim 15,
wherein the joint medical treatment is a medical treatment for a meniscus injury.

17. Synovium-derived mesenchymal stem cell produced by the method according to any one of claims 1 to 14.

18. A cell preparation for a joint medical treatment, produced by the method according to claim 15 or 16.
